# EUROPÄISCHE PATENTSCHRIFT

(11) **EP 2 826 446 B1**
(45) Veröffentlichungstag und Bekanntmachung des Hinweises auf die Patenterteilung: **27.12.2017**
(21) Anmeldenummer: 14172156.3
(22) Anmeldetag: 12.06.2014
(51) Int. Cl.: A61F 2/46, A61F 2/44, A61F 2/30, A61F 2/28

(54) **Bedieninstrument für ein Implantat**
Operating tool for an implant
Instrument d'opération pour un implant

(30) Priorität: 19.07.2013 DE 102013107723
(43) Veröffentlichungstag der Anmeldung: 21.01.2015
(73) Patentinhaber: Böhm, Heinrich, Dr., 99425 Weimar (DE)
(72) Erfinder: Böhm, Heinrich, 99425 Weimar (DE); Burger, Andreas, 78532 Tuttlingen (DE); Widmaier, Gerd, 78532 Tuttlingen (DE); Wenzler, Klaus, 78665 Frittlingen (DE)
(74) Vertreter: Mammel, Ulrike

(56) Entgegenhaltungen:
- EP-A1- 2 476 396
- WO-A2-2011/011609
- WO-A2-2011/011626
- DE-A1-102011 002 076
- US-A1- 2006 069 436
- US-A1- 2011 172 716

## Beschreibung

Die vorliegende Erfindung betrifft ein Bedieninstrument für ein aufspreizbares Implantat, insbesondere für ein spreizbares höhenadaptierbares Implantat für die Lenden- und Brustwirbelsäule, das als Ersatz für Wirbelkörper und/oder Bandscheibenräume bei Versteifungsoperationen dienen kann und ein Bedieninstrument mit diesem Implantat.

Solche lumbalen und thoracalen Implantate werden zur interkorporalen Fusion (Versteifung der Wirbelkörper) eingesetzt. Prinzipiell ist jedoch auch eine intrakorporale Anwendung möglich.

In der Konfiguration als Wirbelkörperersatz kann das Implantat bei Zerstörungen oder Formdefekten jeder Art eingesetzt werden: so bei Tumoren, Wirbelbrüchen, Spätfolgen nach Wirbelbrüchen sowie Infektionen oder angeborenen Fehlbildungen eines oder mehrerer Wirbelkörper.

In der Konfiguration als Wirbelkörperteilersatz kann das Implantat zur intrakorporalen Anwendung als Korrekturinstrument und im Körper verbleibend als Rekonstruktionsimplantat eines Wirbelkörpers angewendet werden.

In der Konfiguration als Zwischenwirbelersatz, "intersomatischer Cage" = "aufspreizbarer Cage", kann das Implantat zur Wiederherstellung der vorderen Säule der Wirbelreihe im Rahmen von Versteifungseingriffen verwendet werden. Hier sind Anwendungsgebiete die degenerativen Wirbelsäulenerkrankungen, Segmentfehlstellungen und Segmentinstabilitäten verschiedenster Ursachen.

Im Allgemeinen wird zunächst der Zwischenwirbelraum vom Rücken beziehungsweise vom Brust- oder Bauchraum her ausgeräumt, bei Wirbelkörperersatz der Wirbelkörper entfernt und dann das Implantat in den entstandenen Zwischenraum eingeführt, um zur Stellungskorrektur sowie als Abstandshalter zur mechanisch stabilen Verbindung zwischen den angrenzenden Wirbelsäulenabschnitten zu dienen.

Es hat sich als vorteilhaft erwiesen, solche Implantate als "Cage", das heißt in Form eines Käfigs, auszubilden, damit in dessen Innenraum beispielsweise Knochenmaterial oder Knochenersatzmaterialien zur Beschleunigung des Einheilens eingebracht werden können. Hierdurch kann die Fusion des Cages ermöglicht bzw. beschleunigt werden.

Über einen Spreizmechanismus nach dem Einbringen in den Wirbelsäulendefekt aufspreizbare Cages haben den Vorteil, technisch einfacher und für die Ankerwirbel schonender implantiert werden zu können: durch das stufenlose Aufspreizen wird das Implantat einerseits fest verklemmt und fixiert. Andererseits erlaubt es individuelle Stellungskorrekturen der angrenzenden Wirbel, um normale geometrische Verhältnisse durch Schaffung von Lordose der Lendenwirbelsäule oder Korrektur einer vermehrten Brustkyphose wieder herzustellen.

Mit Ausnahme des Einsatzes bei bösartigen Tumoren soll das Durchwachsen des Implantats bzw. des rekonstruierten Wirbelsäulenabschnitts mit körpereigenem Knochengewebe ermöglicht werden. Deshalb weisen solche Implantate vorzugsweise Durchbrüche auf.

Abhängig vom Design gewährleisten Cages neben mechanischer Stabilität des Korporektomiedefektes bzw. struktureller Integrität des Zwischenwirbelraumes während des knöchernen Durchbauungsprozesses auch den Schutz des Rückenmarks vor sich verschiebenden Knochenfragmenten.

Ein generelles Problem bei den bekannten Implantaten besteht darin, dass die Implantate nach dem Einbau in die Nachbarwirbel einsinken können, wodurch die Lagebeziehung der Wirbel zueinander und die Gesamtstatik gestört werden. Dies kann insbesondere bei vermindertem Kalksalzgehalt - Osteoporose - der Wirbelkörper auftreten.

Es sind zylinderförmige Implantate bekannt, die an der Zylinderoberfläche ein Schraubgewinde aufweisen. Diese Implantate werden horizontal in den Zwischenwirbelraum eingeschraubt, nachdem zuvor Boden- und Deckplatte der Wirbelkörper mit Bohrinstrumenten arrodiert, das heißt partiell entfernt wurden. Nachteilig an diesen zylinderförmigen Implantaten ist, dass diese eine relativ kleine Auflagezone haben und ein vergleichsweise starkes Einsinken in die Wirbelkörper zeigen und verkanten.

Aus der EP 1 415 622 A1 sind Spreizimplantate mit einer eher quaderförmigen Grundform bekannt, die zwei an einem Ende miteinander verbundene Schenkel aufweisen, wobei die Schenkel mit einem zwischen den Schenkeln angeordneten Schieber aufspreizbar sind, der die Schenkel im aufgespreizten Zustand über ihre gesamte horizontale Breite abstützt und in der Endposition einrastet. Obwohl sich diese Implantate durch eine größere Auflagefläche auf der Endplatte des Nachbarwirbels auszeichnen, ist die Art der Aufspreizung designbedingt sehr begrenzt und immer winkelförmig vorgegeben. Wegen der dann wiederum häufig unvermeidbaren punktförmigen Belastung sinken auch sie oft deutlich in die Nachbarwirbel ein und verlieren so ihre Aufspreiz- bzw. Korrekturfunktion. Darüber hinaus ist designbedingt eine Implantation solcher Cages von der Seite der Wirbelsäule nicht möglich und auf strikt dorsale - selten zusätzlich strikt von vorn kommende - Implantationsrichtung beschränkt. Der Einsatz als Wirbelkörperersatz ist bei beiden vorgenannten Modellen unmöglich.

Aus der US 5,236,460 sind spreizbare teleskopartige Implantate bekannt. Der äußere Implantatkörper, der rohrförmig ist, weist eine axiale Bohrung auf, in der der innere Implantatkörper gleitend geführt ist. Am oberen und unteren Ende der Implantatkörper sind Platten mit Dornen zur Verankerung vorgesehen. Die Spreizung des Implantats erfolgt hydraulisch über eine Flüssigkeit oder ein härtbares Harz. Hierzu weist der äußere Implantatkörper eine Öffnung zum Eintritt des Fluids auf, das dann in eine Kavität im unteren Ende des inneren Implantatkörpers gelangt und diesen axial gleitend nach oben schiebt. Nachteilig hieran ist, dass das Volumen der Spreizkammer nicht für die knöcherne Durchbauung zur Verfügung steht. Das Implantat wird über eine spezielle Klammervorrichtung, die auf die obere Plattform aufgeschraubt wird, mit den Wirbelkörpern verschraubt.

Aus der WO 2009/064787 sind spreizbare Implantate mit einem doppelten teleskopartigen Verstellmechanismus bekannt, die eine obere und eine untere Platte zur Verankerung an den Wirbelstützflächen aufweisen. Der Verstellmechanismus ist über Zahnräder gekoppelt, d.h. eine Rotationsbewegung wird von dem einen auf das andere Zahnrad und somit von dem einen auf den anderen Verstellmechanismus übertragen und so das Implantat aufgespreizt. Die Betätigung soll gemäß dieser Druckschrift durch einen Stab mit Kegelradgetriebe in nicht näher erläuterter Weise erfolgen.

Dieses Implantat ist als Bandscheibenersatz geeignet und einfach und kostengünstig herstellbar. Auch wird infolge der größeren Fläche der oberen und unteren Platte die Gefahr einer sekundären Stellungsveränderung und des Einsinkens minimiert. Nachteilig ist jedoch, dass diese Implantate bislang nicht auf einfache Weise in minimal invasiver Technik eingebracht, aufgespreizt und fixiert werden können. Zudem weisen diese Implantate nicht die erforderliche mechanische Stabilität, vor allem gegen Scherkräfte und die an der Wirbelsäule bei Rumpfbeugung und Streckung immer auftretenden Biegebelastungen auf. Auch ist die natürliche Knochenbildung und die Durchbauung designbedingt bei diesen parallel aufspreizbaren Implantaten erschwert, was zu einer Reduzierung der Langzeitstabilität des Implantats führen kann.

Aus der US 2012/017955 A1 und der EP 2 476 396 A1 ist ein spreizbares Implantat bekannt, das vier Getriebe umfasst, von denen jeweils zwei durch Zahnräder miteinander gekoppelt sind. Ober- und unterhalb der Zahnräder weisen die Getriebe jeweils eine Gewindespindel mit unterschiedlicher Drehrichtung auf, so dass sich bei Betätigung des Zahnrads gleichzeitig das obere und das untere Gewinde aus der oberen und unteren Platte herausschraubt und damit die obere gegenüber der unteren Platte gespreizt wird bzw. bei einem gleichzeitigen Hineinschrauben der Gewinde in die Platte das Implantat komprimiert wird. Das Implantat ist konstruktiv aufwändig und weist bei großem Bauvolumen nur einen geringen Hub auf. Eine knöcherne Durchbauung ermöglicht dieses Implantat nicht. Ein weiterer Nachteil ist die Zylinderform des Implantats mit den kreisförmigen Platten, die zur Verankerung an/in den Wirbelstützflächen dienen. Bei der Positionierung solcher Implantate kommt das Implantat gerade in der Mitte der Wirbelstützfläche zu liegen, somit dem am wenigsten harten Bereich der gesamten Wirbelstützfläche, so dass das Implantat einsinkt. Aufgrund der Zylinderform und des Angriffs des Bedieninstruments in der Zylindermitte kann das Implantat auch nicht mit dem Bedieninstrument bei einer dorsalen Implantation an den Nervenwurzeln vorbei in die richtige Position geschwenkt werden. Für eine dorsale, dorsolaterale oder TLIF-Implantation ist das Implantat somit nicht geeignet. Das Implantat wird mit einem Instrument bedient, das ein Zahnrad und Mittel zur starren Befestigung des Implantats aufweist.

Die DE 10 2011 002 076 A1 lehrt ein Zwischenwirbelimplantat mit einer Vorrichtung zum Einbringen des Implantats, wobei das Zwischenwirbelimplantat mittels eines Gelenks gegenüber der Vorrichtung gedreht und richtig positioniert werden kann.

Die US 2011/0160861 A1 und die US 8,303,663 B2 lehren ein spreizbares Implantat mit einer eher rechteckigen Grundform mit zwei bzw. drei Getrieben. Die Getriebe sind über eine Antriebsstange, die den Boden in Längsrichtung durchquert, gekoppelt. Das Einbringinstrument wird in Längsrichtung an den kurzen Seiten des Implantats angeschraubt, so dass das Implantat insbesondere für eine seitliche, d.h. laterale oder XLIF-Implantation eingesetzt werden kann. Für die TLIF-Technik (Transformaminal/Transarticular Lumbar Interbody Fusion) ist dieses Implantat nicht geeignet, da es nicht um die Nervenwurzeln herum in die optimale Position gebracht werden kann. Bei Anwendung der PLIF-Technik muss bei diesem Implantat über zwei Zugänge implantiert werden. Ein weiterer Nachteil ist, dass das Implantat kein Durchwachsen des Knochens ermöglicht.

Aus der WO 2011/011609 A2 ist ein spreizbares Implantat bekannt, das eine obere und eine untere Platte und drei miteinander gekoppelte Gewinde aufweist.

Die US 2011/172716 A1 lehrt ein spreizbares Implantat mit gekoppelten Gewinden, die unterschiedliche Steigungen aufweisen können.

Die Aufgabe der Erfindung besteht darin, ein im Körper aufspreizbares und dadurch korrigierendes Implantat zur Versteifung des Bandscheibenraumes oder nach Entfernung von Wirbelkörpern als Ersatz von Wirbelkörper(n) und angrenzenden Bandscheiben anzugeben, das mittels eines Bedieninstruments für dieses Implantat einfach handhabbar und bedienbar ist. Das Implantat sollte zudem auf sichere Weise auch in minimal invasiver Technik implantierbar sein und eine Anpassung an die angestrebte optimale Geometrie des Wirbelsäulenabschnitts ermöglichen.

Diese Aufgabe wird durch ein Bedieninstrument mit den Merkmalen des Anspruchs 1 und ein Bedieninstrument mit einem Implantat mit den Merkmalen des Anspruchs 4 gelöst.

Das Implantat umfasst eine obere und eine untere Platte und wenigstens drei Gewinde, die miteinander gekoppelt sind und zum Aufspreizen des Implantats dienen, jedes Gewinde weist jeweils eine Gewindespindel und eine Gewindehülse mit Innengewinde auf, wobei die Steigungen wenigstens zweier Gewinde bzw. zweier jeweils aus Gewindehülse und Gewindespindel bestehender Gewinde unterschiedlich sind.

Hierdurch wird erreicht, dass während des Aufspreizens die Ebene der oberen Platte gegenüber der Ebene der unteren Platte gekippt wird, so dass ein korrigierendes paralleles oder anguläres Aufspreizen der angrenzenden Wirbel , d.h. eine gezielte und definierte Korrektur der Stellung der Nachbarwirbel, erreicht wird.

In einer bevorzugten Ausführungsform sind die Gewinde (in der Draufsicht (d.h. z-Richtung)) nicht linear, sondern auf einem Kreisbogen oder in Form eines "C" angeordnet, d.h. zwei Gewinde liegen endständig und ein Gewinde ist in der Mitte auf dem Kreisbogen angeordnet. Das mittige Gewinde befindet sich gegenüber den beiden endständigen Gewinden somit im hinteren Bereich (d.h. -y-Richtung).

Dadurch, dass in der bevorzugten Ausführungsform die Steigung des mittigen Gewindepaars größer als die der beiden endständigen Gewinde ist, wird beim Aufspreizen der hintere Bereich der oberen Platte, d.h. im Gebiet des mittigen Gewindes, stärker angehoben als der vordere Bereich und so bei hinterkantenparalleler Implantation Lordoseform oder Kyphosekorrektur erreicht. Grundsätzlich sind bei veränderter Implantationsrichtung oder veränderter Gewindesteigung der Einzelzylinder auch Skoliosekorrekturen möglich.

Da die Hauptanwendung jedoch die Korrektur von Kyphose bzw. Rekonstruktion von Lordose ist, wird der Effekt im Folgenden an diesem Beispiel dargestellt.

Das aufgespreizte Implantat in der Lordoseform ist in Bezug auf die obere und untere Platte und die Gewinde spiegelsymmetrisch zur yz-Ebene.

Durch das Zusammenwirken von Gewindehülse und Gewindespindel und den unterschiedlichen Gewindesteigungen lässt sich der erforderliche Abstand zwischen der oberen und unteren Platte in der gewünschten Spreizung in der Lordoseform präzise und auf einfache Weise einstellen.

Selbstverständlich kann der Verstellmechanismus auch mehr als drei Gewindeaufweisen .

Die drei Gewinde sind über Antriebsmittel miteinander gekoppelt. In einer bevorzugten Ausführungsform erfolgt die Kopplung über Zahnräder, die mit den nach außen weisenden Mantelflächen der Gewindehülsen starr verbunden sind.

Durch den dreifachen Verstellmechanismus und die Kopplung wird das Spreizen durch drei Gewindeeingeleitet und so ein Verkanten des Gewindes vermieden. Ein weiterer wesentlicher Vorteil des Verstellmechanismus ist, dass infolge der dreifachen Aufspreizbewegung auch die Fläche der oberen und unteren Platte vergrößert und damit der auf die Wirbelkörper pro Flächenelement wirkende Druck vermindert werden kann. Die Vermeidung punktueller Krafteinleitung in die Ankerwirbel durch anguläres Aufspreizen verringert gegenüber einem parallelen Aufspreizen die Gefahr des unerwünschten Einsinkens des Implantats.

Für die einfache Bedienung und Implantation ist es zudem erforderlich, dass das Bedieninstrument starr an dem Implantat befestigt werden kann. Hierzu weist das Implantat Mittel zur Befestigung, Verschraubung oder sonstigen starren Fixierungen des erfindungsgemäßen Bedieninstruments auf.

Die Verbindung zwischen Implantat und Bedieninstrument muss selbstverständlich lösbar sein. Vorzugsweise umfasst das Bedieninstrument ein Schraubgewinde (Außengewinde), das in einer - entsprechenden Bohrung im Implantat verschraubt werden und das nach der erfolgten Positionierung und Spreizung wieder entfernt werden kann. Die Verbindung kann jedoch auch anderweitig erfolgen.

Wird das Implantat an dem Bedieninstrument befestigt, so wird dabei gleichzeitig auch der Antrieb des Bedieninstruments mit einem der Antriebsräder des Implantats in Eingriff gebracht. Vorzugsweise erfolgt der Antrieb des Bedieninstruments über ein Zahnrad, das über ein weiteres Zahnrad mit einem Schneckenantrieb angetrieben wird.

Diese Ausgestaltung des Bedieninstruments ermöglicht eine schmale schlanke Bauweise auch im proximalen Bereich des Instruments. Dies erlaubt die minimal invasive Implantationstechnik z.B. über tubuläre Retraktoren.

Durch eine Drehbewegung auf den Griff der Getriebestange dreht sich der Schneckenantrieb, der wiederum das weitere Zahnrad antreibt, das infolge der Kopplung das Zahnrad am proximalen Ende des Bedienelements antreibt, das durch die Verschraubung in Eingriff mit dem Zahnrad des Implantats gebracht ist und so die Zahnräder des Implantats und die drei Gewindeantreibt, die das Implantat aufspreizen.

Erfindungsgemäß ermöglicht das Bedieninstrument jedoch nicht nur eine Fixierung des Implantats und ein Aufspreizen desselben, so dass eine lordotische Form erreicht wird, sondern auch, dass eine Schwenkbewegung des Implantats gegenüber dem Handgriff des Bedienelements, d.h., dass das Implantat gegenüber dem (feststehenden) Handgriff in eine seitliche Richtung bewegt werden kann.

Eine solche Einschwenkbewegung des Implantats bei feststehendem Bedienelement erlaubt die Implantationsmöglichkeit von dorsal (PLIF-, TLIF-Technik), da das Implantat mit dem Bedieninstrument mit einem Trokar gerade eingebracht wird und das Implantat dann seitlich um die Nervenstrukturen des Wirbelkanals herumgeführt werden muss.

Diese Einschwenkbewegung wird dadurch erreicht, dass das Befestigungsstück, an dem das Implantat an dem Bedieninstrument festgeschraubt ist, gegenüber dem proximalen Ende des Handgriffs dreh- oder schwenkbar ist. Dies kann dadurch erreicht werden, dass das Befestigungsstück starr mit einem Zahnradabschnitt verbunden ist, der mittels einer Zahnleiste gedreht oder geschwenkt werden kann.

Eine Betätigung der Zahnleiste, die an dem Handgriff des Bedieninstruments befestigt ist, führt somit dazu, dass die Zahnleiste in die Zahnung des Zahnradabschnitts eingreift und diesen etwas dreht/schwenkt, wodurch das mit dem Zahnradabschnitt starr verbundene Implantat ebenfalls aus der ursprünglichen Position seitlich herausgeschwenkt wird.

Dies ermöglichst eine einfache und sichere Handhabung des Implantats während der Operation, da nur ein Bedieninstrument erforderlich ist, das sowohl die Fixierung an dem Bedienelement als auch das Schwenken und das Aufspreizung ermöglicht.

Nach Einbringung in den Zwischenwirbeldefekt oder Korporektomiedefekt erfolgt die Aufspreizung bis zum Erreichen der mechanisch stabilen Verankerung und ggf. Stellungskorrektur. Die erreichte Spreizung ist während der Operation jederzeit veränderbar, um eine etwaige Umpositionierung vorzunehmen oder eine Überspreizung des Wirbelsäulenabschnitts durch Betätigung des Kegelantriebs am Bedieninstrument zurückzufahren. Ohne Betätigung des Schneckengetriebes verbleibt das Implantat jedoch infolge der Reibung sicher in der eingestellten gespreizten Stellung und kann nach Erreichen der gewünschten Spreizung danach durch eine Fixierungsschraube gesichert werden.

Infolge der Stabilität des Implantats durch die dreifachen Säulen ist es möglich, die Fläche, die Winkelgrade und auch die Oberflächenkonfiguration der oberen und unteren Platten relativ frei zu wählen und damit den Formschluss zu optimieren und dem unerwünschten Einsinken der Implantate entgegenzuwirken.

Selbstverständlich kann das Implantat auch drei miteinander gekoppelte Gewinde, wobei die Steigung eines jeden Gewindes von der der anderen beiden Gewinde verschieden ist, aufweisen.

Das Implantat ist vorzugsweise aus Reintitan oder einer Titanlegierung wie Ti₆Al₄, Tantal, Nitinol, einem Kunststoffmaterial wie Polyetheretherketon (PEEK) oder anderen Materialien, die als Implantatwerkstoffe geeignet sind.

Das Implantat wird wie folgt implantiert:

In Bauch-, Seiten- oder Rückenlage wird über einen dorsalen oder ventralen abdominalen oder thorakalen Zugang der Zwischenwirbelraum freigelegt, dann vollständig ausgeräumt. Soll ein Wirbelkörper entfernt werden, wird dies an der zweiten angrenzenden Bandscheibe wiederholt und anschließend die Korporektomie durchgeführt. Entsprechend der Indikation können dabei entweder nur Teile des Wirbelkörpers reseziert werden oder alle Anteile des Wirbelkörpers inklusive von Hinterkante bzw. Knochenfragmenten aus dem Spinalkanal entfernt werden. Dann werden die Endplatten der Ankerwirbel entknorpelt und angeraut, um das Knochenwachstum für den knöchernen Einbau bzw. Durchbau der Hohlräume des Implantats anzuregen.

Anschließend wird das größen- und formadaptiert ausgewählte Implantat mittels des erfindungsgemäßen Bedienelements, einem kombinierten Halte-, Einschwenk-, Aufspreiz- und Arretierungsinstrument, in den ausgeräumten Defekt eingebracht und aufgespreizt. Die Geometrie des Implantats und des Bedieninstruments erlaubt dabei sowohl die Einbringung von vorn, von beiden Seiten als auch von hinten seitlich des Rückenmarkssackes. Der operative Zugang kann konventionell offen, aber auch in minimal invasiver Technik erfolgen. Insbesondere bei der Schlüssellochtechnik erweist sich der Vorteil der symmetrischen Einleitung relativ starker Aufspreiz- und somit Korrekturkräfte.

Da die Endplattengröße und -konfiguration relativ frei mit den zwei Extensionskörpern kombinierbar ist, kann eine große Abstützfläche bei Osteoporose ebenso verwendet werden, wie eine kleine bei solchen Wirbelfrakturen, bei denen Teile des Wirbelkörpers erhalten werden können.

Eine optional an den Innenzylinder angebrachte Kante führt während der Aufspreizung zu einer Anfrischung der nicht zur Abstützung erforderlichen Ankerwirbelendplatte und erlaubt so, dass die jeweils schwer zugängliche Endplatte nicht in einem eigenen Arbeitsgang angefrischt werden muss.

Anschließend erfolgt die Stellungskontrolle mittels intraoperativer Bildgebung, meist mittels Durchleuchtung. Liegt eine suboptimale Implantatlage vor, wird durch Drehen der Getriebestange mit dem Schneckengetriebe in Gegenrichtung das Implantat zusammengefahren, umpositioniert und erneut aufgespreizt.

Das Implantat kann mit Knochen (z.B. Spongiosa) oder anderen Knochenersatz-Materialien (Calciumphosphatpräparate) befüllt werden. Die Rekonstruktion der vorderen Säule ist damit beendet. Die Kombination mit einer dorsalen Stabilisierung wird vor allem im thorako-lumbalen und lumbalen Bereich dringend empfohlen. Je nach Knochenqualität und Blutverlust kann die Mobilisation des Patienten dann am gleichen Tage erfolgen.

Die Erfindung wird nachfolgend anhand eines Ausführungsbeispiels näher beschrieben.

Es zeigen:
Figur 1 eine perspektivische Ansicht des Implantats in maximaler Spreizung,
Figur 2 eine Seitenansicht des Implantats aus Fig. 1 in xz-Richtung,
Figur 3 eine Seitenansicht auf das Implantat aus Figur 1 in yz-Richtung,
Figur 4 das Implantat aus Figur 2, jedoch im ungespreizten Zustand,
Figur 5 eine perspektivische Ansicht des am erfindungsgemäßen Bedieninstrument befestigten und eingeschwenkten Implantats aus Figur 1,
Figur6 erfindungsgemäßes Bedieninstrument mit eingeschwenktem Implantat aus Figur 5 mit Trokar,
Figur 7 Ansicht auf das erfindungsgemäße Bedieninstrument in xy-Richtung von oben (+z-Richtung),
Figur 8 Ansicht auf das erfindungsgemäße Bedieninstrument in xy-Richtung von unten (-z-Richtung) und
Figur 9 den Zahnradabschnitt des erfindungsgemäßen Bedienelements.

Das Implantat 11 umfasst eine obere Platte 12 und eine untere Platte 16, an deren Außenseiten 13, 17 Dorne 14 vorgesehen sind, die zur Verankerung an/in den Wirbelstützflächen dienen.

Obere 12 und untere 16 Platte erstrecken sich jeweils in xy-Richtung und sind voneinander in z-Richtung beabstandet.

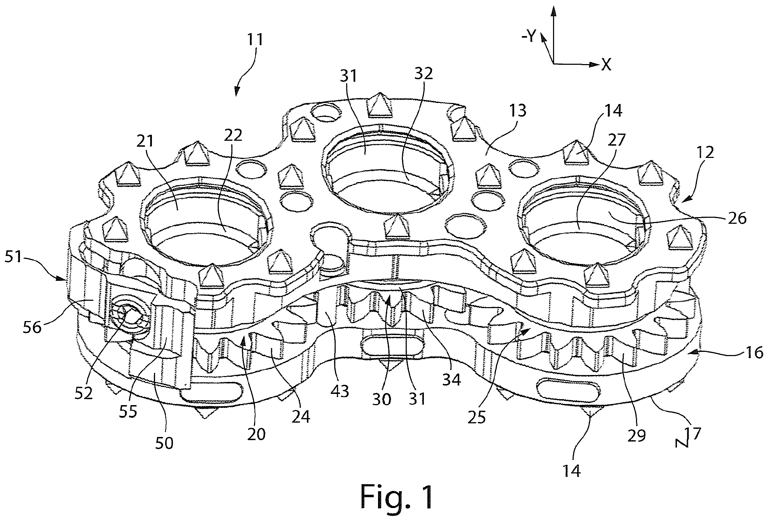

Der Bereich der Platten 12, 16 des Implantats 11, der sich in -y-Richtung, d.h. in Figur 1 "nach hinten" erstreckt, wird -nachfolgend als "hinterer Bereich" und der Bereich, der sich in +y-Richtung erstreckt, als "vorderer Bereich" bezeichnet.

Zwischen der oberen Platte 12 und der unteren Platte 16 befinden sich drei Gewinde20, 25, 30, durch die die untere Platte 16 gegenüber der oberen Platte 12 verschiebbar ist, d.h. das Implantat gespreizt werden kann.

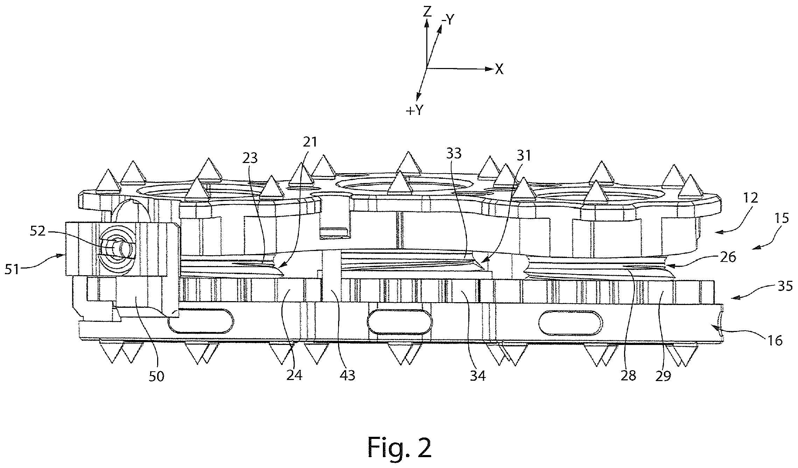

Jedes Gewinde20, 25, 30 besteht aus einer Gewindespindel 21, 26, 31 mit einem Außengewinde 23, 28, 33, das in einer Hülse 22, 27, 32 mit Innengewinde geführt ist (vgl. Fig. 2).

Die Außengewinde 23, 28, 33 der Gewindespindeln 21, 26, 31 sind voneinander beabstandet. Die sich in z-Richtung erstreckenden Längsachsen der Gewindespindeln 21, 26, 31 verlaufen parallel zueinander und sind starr mit der oberen Platte 12 verbunden, d.h. die drei Gewindespindeln 21, 26 und 31 und die obere Platte 12 mit den Dornen 14 bilden das obere Implantatteil 15, das einstückig ist.

Das untere Implantatteil 35 umfasst die untere Platte 16 mit den Dornen 14 sowie die drei Hülsen 22, 27, 32 mit Innengewinden, die drehbar in der Platte 16 gelagert sind.

An den inneren Mantelflächen der Gewindehülsen 22, 27, 32 sind Gewinde vorgesehen, die die Spindelmuttern für die Außengewinde 23, 28, 33 der Gewindespindeln 21, 26, 31 bilden.

Eine jede Gewindehülse 22, 27, 32 weist zudem an ihrer äußeren Mantelfläche ein Antriebsrad 24, 29, 34 auf, das als Zahnrad ausgebildet ist. Die Zahnräder 24, 29, 34 auf den Gewindehülsen 22, 27, 32 koppeln die Rotationsbewegung der drei Gewindehülsen 22, 27, 32.

Die Gewindehülsen 22, 27, 32 sind in der unteren Platte 16 drehbar gelagert. Erfolgt eine Drehbewegung an einem Zahnrad 24 einer Gewindehülse 22, so wird zum einen durch das Ineinandergreifen der Zahnradpaare 24, 34 die Drehbewegung auf die mittlere Gewindehülse 32 und durch die weitere Kopplung des Zahnradpaars 34, 29 auch auf die Gewindehülse 27 übertragen. Diese gekoppelten Zahnräder 24, 29, 34 bewirken, dass der Spreizmechanismus mit einem dreifachen Verstellmechanismus angetrieben wird.

Zum anderen bewirkt die Drehbewegung auf eine jede Gewindehülse 22, 27, 32 jedoch auch, dass eine Verschiebebewegung der Gewindespindeln 21, 26, 31 gegenüber den Gewindehülsen 22, 27, 32 in z-Richtung und damit eine Verschiebung der unteren Platte 16 gegenüber der oberen Platte 12 und damit ein Spreizen des Implantats 11 erfolgt.

Auf diese Weise erfolgt das Aufspreizen des Implantats 11 bei Drehung einer der drei Gewindehülsen 22, 27, 32 in einer gleichmäßigen Bewegung, und ein Verkanten der Gewinde wird vermieden.

Eingeleitet wird die Drehbewegung auf das Zahnrad 24 der Gewindehülse 22 des Gewindes 20 dadurch, dass ein Zahnrad 85 eines anschließend näher beschriebenen Bedieninstruments 80 an dem Zahnrad 24 des Gewindes 20 angreift und die Rotationsbewegung auf das Zahnrad 24 einleitet.

Bei dem Implantat 11 sind die drei Gewinde 20, 25, 30 in der Draufsicht nicht in einer Linie, sondern in Form eines Bogens oder "C" angeordnet. Die beiden Gewinde 20, 25 liegen nahe der Enden des C, das Gewinde 30 liegt in der Mitte des C. Die drei Gewinde 20, 25, 30 sind spiegelsymmetrisch zur yz-Achse. In der Draufsicht (in -z-Richtung) sind die drei Gewinde 20, 25, 30 in Form eines flachen gleichschenkligen Dreiecks angeordnet.

Durch diese Anordnung der Gewinde und die ebenfalls kreisbogenförmige (C-förmige) Form der Platten 12, 16 kann, wenn das mittlere Gewinde 30 eine andere Steigung als die beiden endständigen Gewinde 20, 25 aufweist, während des Spreizens auch eine leichte Kippung der oberen Platte 12 gegenüber der unteren Platte 16 erreicht werden.

Die Steigungen der Außengewinde 23, 28 der Gewindespindeln 21, 26 und die korrespondierenden Innengewinde der Gewindehülsen 22, 27 sind gleich und kleiner als die Steigung des mittleren Gewindes 30, nämlich dem Außengewinde 33 der Gewindespindel 31 und dem korrespondierenden Innengewinde der Gewindehülse 32.

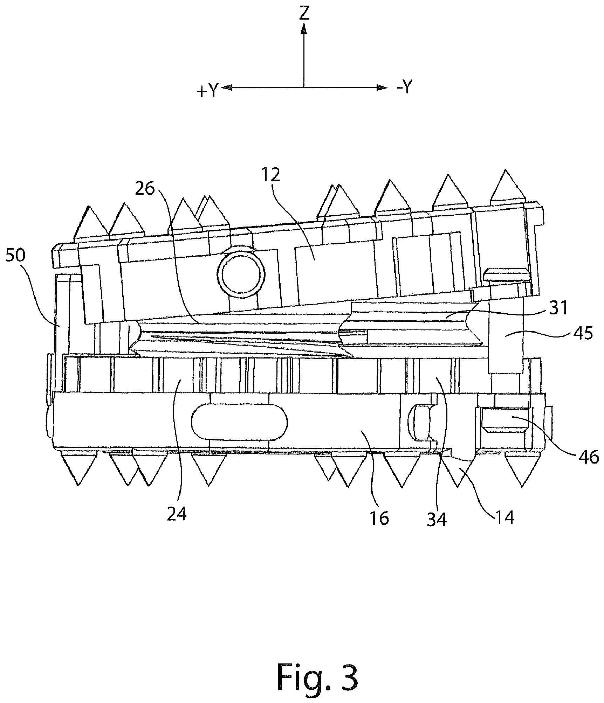

Durch die unterschiedlichen Gewindesteigungen wird die obere Platte 12 beim Spreizen im vorderen Bereich (Bereich in +y-Richtung in Fig. 1) weniger stark angehoben als im hinteren Bereich des Implantats 11 (Bereich in -y-Richtung in Fig. 1), d.h. der Abstand zwischen der oberen Platte 12 und der unteren 16 Platte des Implantats 11 ist am Plattenende in -y-Richtung größer als am Plattenende in +y-Richtung, d.h. die beiden Platten 12, 16 sind geringfügig gegeneinander "gekippt" und weisen somit Lordoseform auf (vgl. insbesondere Fig. 3).

In einer bevorzugten Ausführungsform betragen die Steigungen der Gewinde 23, 22, 28, 27 der beiden äußeren Gewinde20, 25 jeweils 1,0 mm und die Steigung des mittleren Gewinde 30 1,25 mm. Selbstverständlich können die Steigungen jedoch auch anders sein.

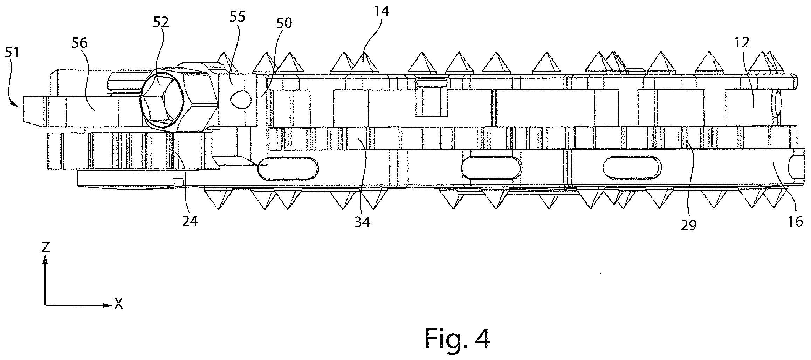

Im nicht gespreizten Zustand (Fig. 4) sind die Abstände in z-Richtung zwischen der oberen Platte 12 und unteren Platte 16 überall gleich, d.h. die Plattenebenen 12, 16 verlaufen parallel zueinander. Parallel zu den Längsachsen (z-Richtung) der Gewindehülsen- und -spindeln 21, 22, 26, 27, 31, 32 ist nahe dem mittleren Gewinde 30 im "hinteren" Bereich (d.h. in -y-Richtung) und im vorderen Bereich (d.h. in +y-Richtung) jeweils ein Stift 43, 45 mit einem Anschlag 44, 46, wobei ein jeder Stift 43, 45 mit Anschlag jeweils eine Bohrung in der oberen Platte 12 und unteren Platte 16 durchquert. Bei maximaler Spreizung liegen die Kopfenden eines jeden Stiftes 43, 45 auf der Oberseite der oberen Platte 12 und die Anschläge 44, 46 auf der Unterseite der unteren 16 Platte auf, d.h. die Stifte 43, 45 und Anschläge 44, 46 begrenzen die Aufspreizbewegung und dienen zudem noch als Führung.

Nachdem die Ausspreizung in +z-Richtung im vorderen Bereich, d.h. in +y-Richtung, geringer ist als im hinteren Bereich", d.h. in -y-Richtung, ist auch der Stift 43 im hinteren Bereich länger als der Stift 45 im vorderen Bereich.

An der unteren Platte 16 sind benachbart dem Gewinde20 zwei sich vertikal erstreckende Abschnitte 50 vorgesehen, die miteinander starr über einen Horizontalabschnitt 51 mit einer Gewindebohrung 52 verbunden sind. Die Gewindebohrung 52 dient zur Befestigung einer am proximalen Ende des Bedieninstruments 80 vorgesehenen Schraube 78.

Untere Platte 16, die beiden vertikalen Abschnitte 50 und der Horizontalabschnitt 51 bilden eine starre Einheit. Die Dicke der oberen und unteren Platte 12, 16 ist relativ gering. Zudem sind die Gewindehülsen 22, 27, 32 und die Gewindespindeln 21, 25, 31 in den Platten 12, 16 befestigt und durchqueren diese, und ein jedes Gewindebesteht genau aus einer Hülse 22, 27, 32 und einer Spindel 21, 25, 31. Hierdurch wird eine kompakte Bauweise mit maximalem Hub erreicht.

Die obere und untere Platte 12, 16 des Implantats können Öffnungen aufweisen, durch die Verankerungsschrauben schräg in die Nachbarwirbel eingedreht werden können.

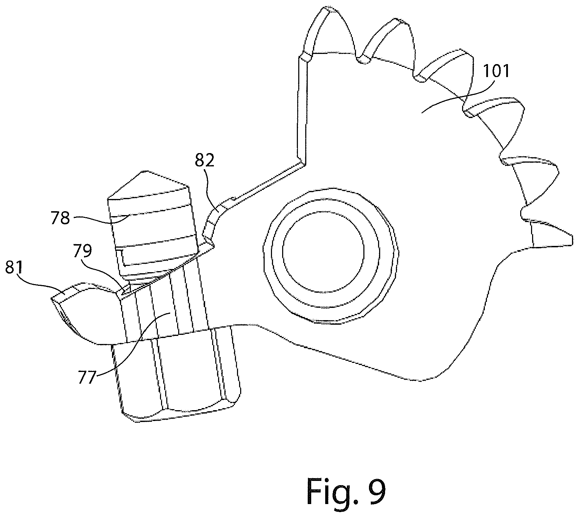

Beidseitig der Gewindebohrung 52 weist der Horizontalabschnitt 51 zwei leicht gewölbte Flächen 55, 56 auf, die zur Positionierung der korrespondierenden Flächen 81, 82 an dem Befestigungsstück 77 (vgl. Fig. 9) am proximalen Ende des Bedieninstruments dienen. Auf diese Weise wird das Implantat bei fixierter Schraube starr mit dem Bedieninstrument verbunden.

Die Stirnflächen der Gewindehülsen 22, 27, 32 und hülsenförmigen Gewindespindeln 21, 26, 31 sind offen, d.h. nicht von der oberen Platte 12 bzw. unteren Platte 16 abgedeckt. Das Implantat 11 weist somit drei durchgängige, sich von der Außenseite 13 der oberen Platte 12 bis zur Außenseite 17 der unteren Platte 16 erstreckende rohrförmige Öffnungen auf.

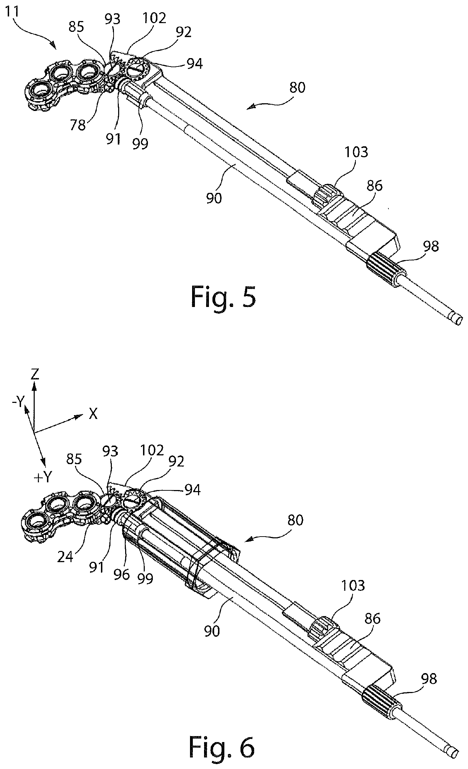

Das Implantat wird mit einem Instrument 80 bedient, das in den Figuren 5 ff. näher dargestellt ist. Das Instrument 80 dient sowohl zum Befestigen, als auch zum Eindrehen bzw. Einschwenken und Einbringen des Implantats 11, als auch zum Aufspreizen und zur Fixierung der Spreizstellung.

Um das Implantat 11 für die spätere Handhabung, d.h. Einbringen, Positionieren, Einschwenken und Aufspreizen, sicher an dem Bedieninstrument 80 zu befestigen, wird das Instrument 80 mittels einer Feststellschraube 78 am proximalen Ende des Bedieninstruments 80 in die Gewindebohrung 52 des Horizontalabschnitts 51 des Implantats 11 geschraubt.

Die Feststellschraube 78 ist in einem Schraubgewinde 79 in einem Befestigungsstück 77 unverlierbar befestigt. Das Befestigungsstück 77 ist starr mit dem später noch detaillierter beschriebenen Zahnradabschnitt 101 verbunden (Fig. 9).

Die dem Schraubgewinde 79 benachbarten Positionierflächen 81, 82 korrespondieren zu den dem Schraubgewinde 52 benachbarten Positionierflächen 55, 56, so dass eine formschlüssige, starre Befestigung des Implantats 11 am Bedieninstrument 80 erreicht wird.

Durch die Verschraubung des Implantats 11 mit dem proximalen Ende des Bedieninstruments 80 wird nicht nur eine starre Verbindung zwischen Implantat 11 und Bedieninstrument 80 und damit ein präzises Einbringen erreicht, sondern gleichzeitig auch das sich am proximalen Ende des Bedieninstruments 80 befindliche Zahnrad 85 in Eingriff mit dem Zahnrad 24 des Implantats gebracht. Dabei liegen die Zahnräder 85, 24 in einer Ebene (xy-Ebene), und die Zahnung des Rads 85 greift seitlich in die Zahnung des Antriebsrads 24 ein.

Die Drehbewegung des Zahnrads 85 erfolgt durch Drehung des distalen Endes einer Getriebestange 90, an deren proximalen Ende sich ein Schneckenantrieb 91 befindet, dessen Drehbewegung mittels eines weiteren Zahnrads 92 auf das Zahnrad 85, das das Zahnrad 24 des Implantats 11 antreibt, übertragen wird.

Die Zahnräder 85, 92 liegen in einer Ebene. Der Schneckenantrieb 91 greift seitlich an der nach außen weisenden Zahnung des Zahnrads 92 an.

Durch diese Anordnung kann bei einer schlanken Bauweise auch des proximalen Endes des Bedieninstruments 80 eine Rotationbewegung des Zahnrads 85 am proximalen Ende eingeleitet werden.

Die Getriebestange 90 ist dabei drehbar in dem Handstück 86 gelagert, und zwar derart, dass die Getriebestange 90 jeweils in einer Aufnahme 96, 97 in dem Handstück 86 nahe dem proximalen und dem distalen Ende der Stange 90 aufgenommen ist. Durch einen gegenüber den Aufnahmen 96, 97 größeren Umfang des Schneckenantriebs 91, einen Anschlag 99 nahe dem proximalen Ende des Bedieninstruments 80 und den Griff 98 am distalen Ende ist die Stange 90 gegen das Herausfallen gesichert.

Die Zahnräder 85, 92 sind mit Schrauben 93, 94 in dem Handstück 86 drehbar gelagert. Das Zahnrad 92 ist weitaus größer als das Zahnrad 85.

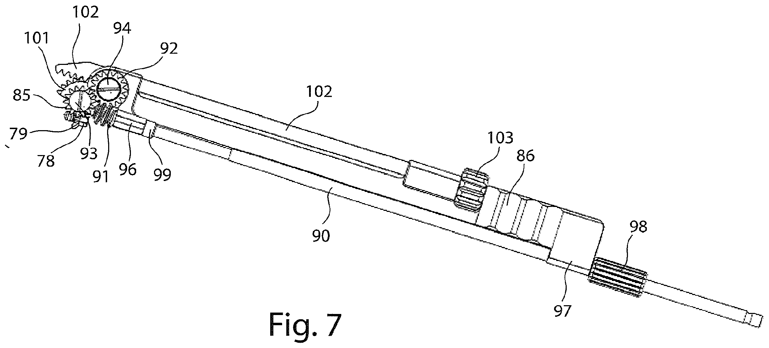

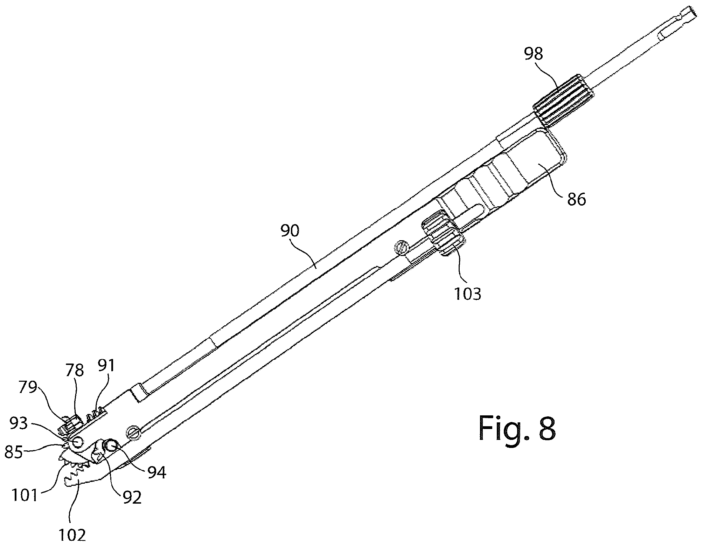

In der in Fig. 7 dargestellten Draufsicht liegt der Kopf der Schraube 93 oberhalb des Zahnrads 85. Der Schaft der Schraube 93 durchquert eine Bohrung in dem Zahnrad 85. Unterhalb des Zahnrads 85 ist ein Zahnradabschnitt 101 vorgesehen, der ebenfalls von dem Gewinde der Schraube 93 durchquert wird (vgl. Fig. 8).

Wird der Zahnradabschitt 101 gedreht, so wird der Abschnitt 101 mit dem damit verbundenen Befestigungsstück 77 nebst Schraubgewinde 79 auf einer Kreisbahn um die Drehachse des Zahnrads 85, d. h. den Schaft der Schraube 93, gedreht. Ein mit dem Bedieninstrument 80 fest verschraubtes Implantat 11 kann somit bei Betätigung des Zahnradabschnitts 101 auf einer Kreisbahn um das Zahnrad 85 geschwenkt werden. Anders ausgedrückt wird bei einer Drehbewegung auf den Zahnradabschnitt 101 das Implantat 11 am proximalen Ende des Bedieninstruments 80 um das Zahnrad 85 bzw. die Schraube 93 geschwenkt.

Die Drehbewegung des Zahnradabschnitts 101 erfolgt über eine Zahnstange 102, die in dem Handstück 86 gehalten ist und die über eine Rändelschraube 103 vor und zurück bewegt werden kann.

Eine Vorwärtsbewegung der Zahnstange 102 bewirkt eine Vorwärtsbewegung des Zahnradabschnitts 101 und damit eine Drehbewegung auf den Zahnradabschnitt 101 und damit ein Schwenken des Implantats 11.

Die Längsachsen der Stange 90, der Zahnstange 102 und des Handstücks 86 verlaufen parallel zueinander.

## Patentansprüche

1. Bedieninstrument (80) für ein Implantat (11), welches Bedieninstrument (80) einen Schneckenantrieb (91) oder ein Zahnrad (85, 92) zum Antrieb des Antriebsrads (24) des Implantats (11) umfasst und Mittel (78, 79, 81, 82) zur starren Befestigung des Implantats (11), **dadurch gekennzeichnet, dass** die Befestigungsmittel (78,79, 81, 82) starr mit einem drehbaren Zahnradabschnitt (101) verbunden sind und der drehbare Zahnradabschnitt (101) mittels einer Betätigungsstange (102) angetrieben wird, wobei die Befestigungsmittel (78, 79, 81, 82) gegenüber der Längsachse des Bedieninstruments (80) einschwenkbar sind, so dass ein an dem Bedieninstrument (80) befestigtes Implantat während der Operation gegenüber der Längsachse des Bedieninstruments (80) geschwenkt werden kann.

2. Bedieninstrument (80) nach Anspruch 1, **dadurch gekennzeichnet, dass** der Schneckenantrieb (91) ein erstes Zahnrad (92) und dieses Zahnrad (92) ein zweites Zahnrad (85) antreibt und die Zahnräder (85, 92) in einer Ebene liegen.

3. Bedieninstrument (80) nach einem der Ansprüche 1 oder 2, **dadurch gekennzeichnet, dass** der Antrieb der Betätigungsstange (102) über eine Rändelschraube (103) erfolgt.

4. Bedieninstrument nach einem der Ansprüche 1 bis 3 mit einem spreizbaren Implantat (11) mit einer oberen Platte (12) und einer unteren Platte (16), die sich in der xy-Ebene erstrecken, die zur Verankerung an/in den Wirbelstützflächen dienen und wenigstens drei Gewinde (20, 25, 30), die miteinander gekoppelt sind, wobei die Gewinde (20, 25, 30) zum Aufspreizen des Implantats (11) dienen und jedes Gewinde (20, 25, 30) eine Gewindespindel (21, 26, 31) und eine Gewindehülse mit korrespondierendem Innengewinde (22, 27, 32) aufweist, wobei die Steigung eines Gewindes (30) von der Steigung der anderen Gewinde (20, 25) verschieden ist.

5. Bedieninstrument mit einem spreizbaren Implantat (11) nach Anspruch 4, **dadurch gekennzeichnet, dass** die Gewinde (20, 30, 25) in der Draufsicht (z-Richtung) auf einem Kreisbogen oder C-förmig angeordnet sind.

6. Bedieninstrument mit einem spreizbaren Implantat (11) nach einem der Ansprüche 4 oder 5, **dadurch gekennzeichnet, dass** zwei endständige Gewinde (20, 25) und ein mittiges Gewinde (30) vorgesehen sind und die beiden endständigen Gewinde (20, 25) eine andere, vorzugsweise geringere Steigung, aufweisen als das mittige Gewinde (30).

7. Bedieninstrument mit einem spreizbaren Implantat (11) nach einem der Ansprüche 4 bis 6, **dadurch gekennzeichnet, dass** an jeder Gewindehülse (22, 27, 32) Antriebsräder (24, 29, 34) vorgesehen sind, die miteinander gekoppelt sind.

8. Bedieninstrument mit einem spreizbaren Implantat (11) nach einem der Ansprüche 4 bis 7, **dadurch gekennzeichnet, dass** das Implantat (11) einen Abschnitt (51) mit einer Gewindebohrung (52) zur Verschraubung des Bedieninstruments (80) aufweist.

## Claims

1. Operating instrument (80) for an implant (11), which operating instrument (80) comprises a worm drive (91) or a toothed wheel (85, 92) for driving the drive wheel (24) of the implant (11), and means (78, 79, 81, 82) for rigidly securing the implant (11), **characterized in that** the securing means (78, 79, 81, 82) are rigidly connected to a rotatable toothed wheel portion (101), and the rotatable toothed wheel portion (101) is driven by means of an actuation rod (102), wherein the securing means (78, 79, 81, 82) are pivotable with respect to the longitudinal axis of the operating instrument (80), such that an implant secured on the operating instrument (80) can be pivoted with respect to the longitudinal axis of the operating instrument (80) during the operation.

2. Operating instrument (80) according to Claim 1, **characterized in that** the worm drive (91) drives a first toothed wheel (92), and this toothed wheel (92) drives a second toothed wheel (85), and the toothed wheels (85, 92) lie in one plane.

3. Operating instrument (80) according either of Claims 1 and 2, **characterized in that** the actuation rod (102) is driven via a knurled screw (103) .

4. Operating instrument according to one of Claims 1 to 3 with an expandable implant (11) with an upper plate (12) and a lower plate (16) which extend in the xy plane, which serve for anchoring on/in the vertebral support surfaces, and at least three threads (20, 25, 30) which are coupled to each other, wherein the threads (20, 25, 30) serve to expand the implant (11), and each thread (20, 25, 30) has a threaded spindle (21, 26, 31) and a threaded sleeve with corresponding inner thread (22, 27, 32), wherein the pitch of one thread (30) is different from the pitch of the other threads (20, 25).

5. Operating instrument with an expandable implant (11) according to Claim 4, **characterized in that** the threads (20, 30, 25), in a plan view (z direction), are arranged on an arc of a circle or in a C shape.

6. Operating instrument with an expandable implant (11) according to one of Claims 4 or 5, **characterized in that** two end threads (20, 25) and a central thread (30) are provided and the two end threads (20, 25) have a different pitch, preferably a smaller pitch, than the central thread (30).

7. Operating instrument with an expandable implant (11) according to one of Claims 4 to 6, **characterized in that** drive wheels (24, 29, 34) are provided on each threaded sleeve (22, 27, 32) and are coupled to each other.

8. Operating instrument with an expandable implant (11) according to one of Claims 4 to 7, **characterized in that** the implant (11) has a portion (51) with a threaded bore (52) for screwing on the operating instrument (80).

## Revendications

1. Instrument de manipulation (80) pour un implant (11), lequel instrument de manipulation (80) comprend un entraînement par vis sans fin (91) ou une roue dentée (85, 92) permettant d'entraîner la roue d'entraînement (24) de l'implant (11) ainsi que des moyens (78, 79, 81, 82) permettant de fixer de manière rigide ledit implant (11), **caractérisé en ce que** les moyens de fixation (78, 79, 81, 82) sont reliés de manière rigide à un segment de roue dentée rotatif (101) et que ledit segment de roue dentée rotatif (101) est entraîné au moyen d'une tige d'actionnement (102), les moyens de fixation (78, 79, 81, 82) pouvant pivoter par rapport à l'axe longitudinal de l'instrument de manipulation (80) de sorte qu'il est possible, pendant l'opération, de faire pivoter un implant fixé sur l'instrument de manipulation (80) par rapport à l'axe longitudinal dudit instrument de manipulation (80).

2. Instrument de manipulation (80) selon la revendication 1, **caractérisé en ce que** l'entraînement par vis sans fin (91) entraîne une première roue dentée (92) et que ladite roue dentée (92) entraîne une deuxième roue dentée (85) et que lesdites roues dentées (85, 92) se situent sur un même plan.

3. Instrument de manipulation (80) selon l'une des revendications 1 ou 2, **caractérisé en ce que** l'entraînement de la tige d'actionnement (102) a lieu par l'intermédiaire d'une vis moletée (103).

4. Instrument de manipulation selon l'une quelconque des revendications 1 à 3 avec un implant expansible (11) pourvu d'une plaque supérieure (12) et d'une plaque inférieure (16) qui s'étendent dans un plan x,y et qui servent à l'ancrage sur/dans les surfaces d'appui des vertèbres, ainsi que d'au moins trois filets (20, 25, 30) qui sont couplés entre eux, lesdits filets (20, 25, 30) servant à écarter l'implant (11) et chaque filet (20, 25, 30) présentant une tige filetée (21, 26, 31) et un manchon fileté à filetage intérieur correspondant (22, 27, 32), le pas d'un filet (30) étant différent du pas des autres filets (20, 25).

5. Instrument de manipulation avec un implant expansible (11) selon la revendication 4, **caractérisé en ce que** les filets (20, 30, 25) sont disposés, en vue par-dessus (en direction z), sur un arc de cercle ou en forme de C.

6. Instrument de manipulation avec un implant expansible (11) selon l'une des revendications 4 ou 5, **caractérisé en ce qu'**il est prévu deux filets terminaux (20, 25) et un filet central (30) et que les deux filets terminaux (20, 25) présentent un autre pas de filetage que le filet central (30), de préférence un pas de filetage inférieur à ce dernier.

7. Instrument de manipulation avec un implant expansible (11) selon l'une quelconque des revendications 4 à 6, **caractérisé en ce que** sur chaque manchon fileté (22, 27, 32) sont prévues des roues d'entraînement (24, 29, 34) qui sont couplées entre elles.

8. Instrument de manipulation avec un implant expansible (11) selon l'une quelconque des revendications 4 à 7, **caractérisé en ce que** l'implant (11) présente une partie (51) pourvue d'un trou fileté (52) destiné au vissage de l'instrument de manipulation (80).
